Europäisches Patentamt

European Patent Office

Office européen des brevets

(19)

(11) **EP 0 893 092 A1**

(12) ## DEMANDE DE BREVET EUROPEEN

(43) Date de publication:
**27.01.1999 Bulletin 1999/04**

(51) Int Cl.⁶: **A61B 3/02**, A61B 3/06

(21) Numéro de dépôt: **98480047.4**

(22) Date de dépôt: **17.07.1998**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **22.07.1997 MC 2382**

(71) Demandeurs:
• **EXSYMOL S.A.M.**
**MC-98000 Monte Carlo (MC)**
• **BABIZHAYEV, Marc**
**74, Moscou, 127434 (RU)**

(72) Inventeurs:
• **Seguin, Marie-Christine**
**98000 Monaco (MC)**
• **Babizhayev, Mark A.**
**127434 Moscou (RU)**

(74) Mandataire: **Bonneau, Gérard**
**Cabinet Bonneau,**
**Conseil en Propriété Industrielle,**
**7, Avenue Gazan**
**06600 Antibes (FR)**

(54) **Procédé d'évaluation de dysfonctionnements oculaires et dispositifs de mise en oeuvre de ce procédé**

(57) Procédé d'évaluation de dysfonctionnements oculaires, caractérisé en ce qu'on place l'oeil (1) d'un patient, à une distance prédéfinie d'une source de lumière éblouissante (3), de manière à ce qu'il visualise une cible (4) placée à proximité de la source de lumière éblouissante (3), on approche la cible (4) de la source (3) jusqu'à ce que le patient ne puisse plus distinguer la cible (4) de la source (3), on éloigne ensuite lentement la cible (4) de la source (3), jusqu'à l'instant précis où le patient distingue à nouveau la cible (4) et, à cet instant, on mesure l'angle ω la lumière incidente entre la source (3) et la cible (4).

FIG. 1

## Description

L'invention a pour objet un nouveau procédé d'évaluation de dysfonctionnements oculaires, l'application de ce procédé pour l'évaluation des opacités des milieux oculaires et pour le contrôle des fonctions rétiniennes, et des dispositifs de mise en oeuvre de ce procédé.

Pour évaluer la perte ou la faiblesse de certaines fonctions visuelles dont se plaignent les patients atteints de troubles de la vue, on mesure généralement l'acuité visuelle. Toutefois, cette mesure n'est pas toujours suffisante, notamment pour répondre au besoin des patients souffrant de cataracte, par exemple, qui se plaignent de troubles issus de la détérioration de la sensibilité au contraste dans des conditions de lumière éblouissante.

Il semble que la distorsion visuelle provoquée par la cataracte soit due à une augmentation de la diffusion de la lumière, résultant des opacités présentes dans les milieux oculaires. Par milieu oculaire, on entend la cornée, l'humeur vitreuse et le cristallin.

Si l'on sait mesurer en clinique l'acuité visuelle, on sait également réaliser des tests de sensibilité au contraste. Toutefois, ces tests ne permettent pas d'évaluer précisément l'évolution d'une cataracte. Ils ne permettent pas non plus d'augurer des résultats de l'opération de la cataracte, et surtout, ils ne permettent pas de distinguer, dans la pathologie, la contribution due à la dégénérescence rétinale de celle due aux opacités des milieux oculaires. Enfin, les tests de sensibilité au contraste, de l'art antérieur, n'ont pas une reproductibilité fiable.

De plus, comme la perte d'acuité visuelle, la perte de sensibilité au contraste n'est pas spécifique de cette pathologie. Ainsi, si un patient souffre de la cataracte et simultanément d'une autre pathologie des fonctions visuelles, le clinicien ne peut pas savoir, à partir des tests de l'art antérieur, dans quelle mesure la perte de sensibilité au contraste ou d'acuité visuelle, est due à la cataracte ou à l'autre pathologie.

Pour notamment remédier à ces inconvénients, un des buts de la présente invention et de proposer un nouveau procédé d'évaluation de dysfonctionnements oculaires, qui permette notamment de diagnostiquer une cataracte, de quantifier son évolution et les résultats dus à l'administration de médicaments anti-cataracte, ou de prévoir le résultat d'une future opération de la cataracte.

En effet, le procédé selon l'invention permet de mesurer la vision de l'axe central de l'oeil et de distinguer d'une part les troubles dus aux opacités se trouvant sur l'axe central de la vision, et d'autre part les troubles dus aux opacités se trouvant dans le reste du milieu oculaire.

Le procédé selon l'invention est caractérisé en ce qu'on place l'oeil d'un patient à une distance prédéfinie d'une source de lumière éblouissante, de manière à ce qu'il visualise une cible placée à proximité de la source de lumière éblouissante, puis l'on approche la cible de la source jusqu'à ce que le patient ne puisse plus distinguer la cible de la source, on éloigne ensuite lentement la cible de la source, jusqu'à l'instant précis où le patient distingue à nouveau la cible et, à cet instant, on mesure l'angle de la lumière incidente entre la source et la cible.

De préférence, la distance prédéfinie entre la source de lumière et l'oeil du patient est 300 mm.

Avantageusement, la source de lumière éblouissante est une lampe halogène ou une lampe à incandescence.

De préférence, la cible est lumineuse.

Suivant un mode de réalisation particulier de l'invention, la cible est une source de lumière de couleur rouge ou verte.

De préférence, on modifie artificiellement le contraste sur la cible.

Suivant un autre mode de réalisation particulier de l'invention, la source et la cible sont dans le même plan tangentiel vertical et, dans ce cas, la mesure de l'angle de la lumière incidente entre la source et la cible s'effectue en mesurant la distance entre la source et la cible.

Le procédé selon l'invention est avantageusement appliqué pour l'évaluation des opacités des milieux oculaires.

Il est également avantageusement utilisé pour contrôler les fonctions rétiniennes.

Un autre objet de l'invention est de proposer un dispositif de mise en oeuvre de ce procédé, caractérisé en ce qu'il comprend, à hauteur de l'oeil du patient, une source de lumière éblouissante et une cible susceptibles de coulisser sur une réglette.

De préférence, le dispositif selon l'invention comprend en outre un tube conique ou un tube cylindrique destiné à canaliser le champ de vision du patient.

Encore un objet de l'invention est de proposer un dispositif de mise en oeuvre de ce procédé, caractérisé en ce qu'il est adapté sur un dispositif ophtalmologique de mesure de l'acuité visuelle soit simplement hémisphérique, soit à périmètre hémisphérique.

De préférence, le dispositif ophtalmologique de mesure de l'acuité visuelle à périmètre hémisphérique sur lequel est adapté le dispositif selon l'invention comprend un logiciel de mesure.

La description qui suit se lit au regard des deux figures jointes, et illustre non limitativement l'invention.

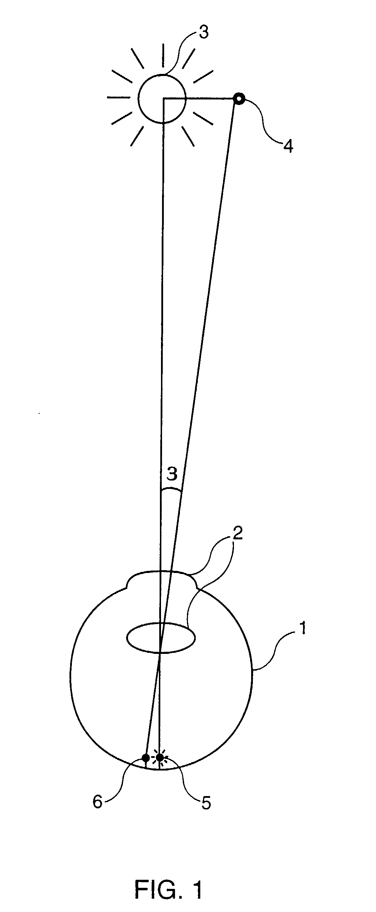

La figure 1 représente schématiquement le procédé selon l'invention, $\omega$ étant l'angle de la lumière incidente entre la source et la cible.

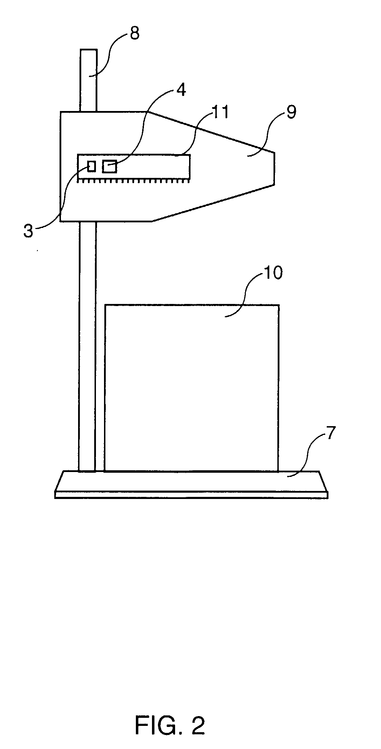

La figure 2 représente une vue schématique de face d'un dispositif particulier de réalisation du dispositif de mise en oeuvre du procédé selon l'invention.

Pour fournir de bonnes indications sur l'évaluation de la cataracte, un test doit répondre à deux critères : refléter la perte des fonctions visuelles et ne pas être lié à la mesure de l'acuité visuelle.

Les patients atteints par la cataracte se plaignent souvent d'une perte de leur capacité à voir des objets vivement éclairés par la lumière du soleil et d'être fortement éblouis par les phares des voitures arrivant en face, lors de la conduite de nuit.

Le procédé selon l'invention est basé sur l'observation suivante : puisque la cataracte dégrade le contraste perçu par l'oeil 1 du patient, un moyen pour l'évaluer est de modifier le contraste sur une cible 4, de préférence lumineuse, placée à proximité d'une source de lumière éblouissante 3, et de mesurer l'angle ω de la lumière incidente entre la source 3 et la cible 4, à l'instant où le patient peut reconnaître la cible 4. Cet angle ω correspond à la projection de la source 3 sur la rétine de l'oeil 1 du patient.

Le fait d'utiliser une source de lumière éblouissante 3 imite le passage de la lumière du soleil dans le milieu oculaire 2.

Le procédé selon l'invention peut être réalisé de la manière suivante : le patient est placé dans une pièce plongée dans la pénombre. Le cas échéant, il est soumis à la meilleure correction visuelle que nécessite son état (lunettes, lentilles).

Un seul de ses deux yeux 1 est examiné, et l'autre oeil est masqué par un cache opaque. Le patient est positionné en face du dispositif, de telle sorte que son oeil 1 soit à une distance prédéfinie d'une source de lumière éblouissante 3, de préférence à 300 mm de cette source 3.

Pour l'exactitude de la mesure, il est important que l'oeil 1 du patient soit bien en face du dispositif. Suivant un mode de réalisation particulier de l'invention, le dispositif peut être équipé avec un tube conique ou cylindrique, de préférence opaque, permettant de canaliser le champ de vision du patient.

Suivant un mode de réalisation particulier de l'invention, la source de lumière éblouissante 3 est une lampe halogène ou une simple lampe à incandescence.

A proximité de la source 3, mais à une distance suffisante pour que le patient, malgré l'éblouissement due à la source de lumière éblouissante 3, la distingue, on place une cible 4. De préférence, la cible 4 est lumineuse.

Suivant un mode de réalisation particulier de l'invention, la cible 4 est constituée par un ou plusieurs chiffre(s) ou signe(s) lumineux. Suivant un mode de réalisation préféré de l'invention, la cible est constituée de cercles de Landolt, qui sont particulièrement adaptés pour mesurer l'acuité visuelle et la sensibilité au contraste.

Suivant un mode de réalisation particulier de l'invention, la surface de la cible est d'environ 5 mm$^2$.

L'opérateur approche la cible 4 de la source 3 jusqu'à ce que le patient ne puisse plus distinguer la cible 4 de la source 3. Pour éviter les artefacts dus à la persistance rétinienne, aucune mesure n'est prise à ce moment-là.

L'opérateur éloigne ensuite lentement la cible 4 de la source de lumière éblouissante 3, jusqu'au moment où le patient est capable de distinguer à nouveau la cible 4. Ce moment précis est indiqué par le patient à l'opérateur et constitue la valeur-seuil. Ce dernier mesure alors l'angle ω de la lumière incidente entre la source 3 et la cible 4.

L'indice I de la lumière diffusée répond à l'équation :

$$I = I_0 \cos^2 \omega$$

dans laquelle :

$I_0$ est l'intensité maximale de la lumière
ω est l'angle de la lumière incidente entre la source et la cible.

L'angle de la lumière incidente entre la source 3 et la cible 4 peut être mesuré de différentes manières : suivant un premier mode de réalisation de l'invention, la cible 4 et la source 3 sont dans un même plan vertical, tangentiel par rapport à la lumière émise. Dans ce cas, pour mesurer l'angle ω de la lumière incidente entre la source 3 et la cible 4, il suffit d'en mesurer la projection (5,6) sur ce plan vertical, ce qui revient à mesurer la distance entre la source 3 et la cible 4.

Suivant un autre mode de réalisation de l'invention, la cible 4 et la source de lumière éblouissante 3 ne sont pas dans le même plan vertical, auquel cas l'angle ω de la lumière incidente entre la source 3 et la cible 4 peut être mesuré par tout moyen approprié, notamment par un logiciel de calcul approprié.

Comme on sait que la diffusion de la lumière par les milieux oculaires 2 est fonction de l'opacité du cristallin, cette mesure permet de calculer le rayon de la lumière diffusée et donc la sensibilité du patient à l'éblouissement. Cette mesure peut être utilisée, dans le cadre d'une série d'évaluations chez un même patient, comme index relatif de la transparence du milieu oculaire 2 examiné. On peut ainsi notamment mesurer les opacités de l'humeur vitreuse.

Un autre avantage du procédé selon l'invention est qu'il permet de mesurer des modifications très légères dans l'opacité du milieu oculaire 2 examiné, qui se traduisent notamment par une variation de la valeur-seuil, et d'apporter une précision accrue par rapport aux procédés et dispositifs de l'art antérieur.

Suivant un mode de réalisation particulier de l'invention, on utilise comme cible 4 une source de lumière de couleur rouge ou verte, ce qui permet d'évaluer la longueur d'onde de la lumière diffusée. De préférence, le flux lumineux de la source verte est de 0,75 lux, le flux lumineux de la source rouge est de 1,7 lux et le flux lumineux de la source de lumière éblouissante est de 1000 lux ; le rapport des flux lumineux de la source de lumière éblouissante sur la source verte est d'environ 1333 ; le rapport des flux lumineux de la source de lumière éblouissante sur la source rouge est d'environ 588.

Lorsqu'on utilise un tube conique ou cylindrique destiné à canaliser le champ de vision du patient, il est possible d'utiliser des valeurs de flux lumineux bien moindres.

Avantageusement, les différentes sources de lumières sont équipées de moyens permettant de faire varier leur flux lumineux.

Les valeurs préférées du flux lumineux sont susceptibles de varier en fonction de l'utilisation du dispositif selon l'invention, et notamment de son adaptation éventuelle sur un dispositif à périmètre hémisphérique.

Le procédé selon l'invention peut faire l'objet d'un dispositif de mise en oeuvre spécifique dans lequel la source 3 et la cible 4 sont positionnés sur une réglette 11, sur laquelle ils coulissent, la source éblouissante 3 pouvant être positionnée à droite ou à gauche de la cible 4. De préférence, la réglette est horizontale.

De préférence, le dispositif selon l'invention comprend une base 7, sur laquelle est fixée l'extrémité d'une barre verticale 8 qui porte un boîtier 9 mobile le long de la barre 8. Le boîtier 9 soutient une réglette 11 horizontale sur laquelle peut coulisser la cible 4 et/ou la source de lumière éblouissante 3.

La source de lumière éblouissante 3 et la cible 4 sont alimentées par une source d'alimentation électrique 10.

Suivant un mode de réalisation de l'invention, le procédé selon l'invention peut être adapté sur un appareil à périmètre hémisphérique servant à mesurer habituellement l'acuité visuelle. Ce type d'appareil est déjà communément employé par les ophtalmologues pour leurs essais cliniques de routine.

Suivant un autre mode de réalisation de l'invention, le procédé selon l'invention peut être adapté sur un appareil hémisphérique du type "B.A.T." (Brightness Acuity Tester) commercialisé par la société américaine MENTOR O&O Inc. Le B.A.T. présente une source d'éblouissement hémisphérique : il s'agit par exemple d'une boule hémisphérique d'un diamètre de 60 mm avec une ouverture (centrale) de 12 mm, à distance de laquelle le patient place son oeil. Le patient va pouvoir distinguer plusieurs sources de lumière, qui apparaissent à des points différents de l'hémisphère.

Il convient de souligner que le B.A.T. n'est pas un dispositif à périmètre et, à ce titre, peut être utilisé pour mesurer la vision de l'axe central de l'oeil du patient mais n'est pas susceptible de mesurer la vision périphérique.

Toutefois, un inconvénient du B.A.T. (Mentor O&O Inc.) est que les sources d'éblouissement apparaissent sur l'hémisphère entier ce qui diminue la sensibilité au contraste de l'oeil.

Suivant le mode de réalisation préféré de l'invention, la source d'éblouissement est ponctuelle (et non hémisphérique) et dans l'axe central de la vision du patient, ce qui permet d'évaluer la contribution uniquement des régions opacifiées dans le milieu oculaire qui diminue le contraste.

Suivant un mode de réalisation particulier de l'invention, l'une au moins des sources lumineuses peut être émise et contrôlée par ordinateur. De préférence, la source lumineuse concernée sera la cible parce que l'intensité d'illumination habituellement délivrée par les ordinateurs est trop faible pour créer l'éblouissement nécessaire à la source éblouissante. La source de lumière éblouissante est donc avantageusement séparée de l'ordinateur.

Toutefois, un dispositif de réflexion d'une source extérieure par un miroir contrôlé par l'ordinateur peut être avantageusement installé : ce mode de réalisation présente l'avantage de permettre à l'opérateur de contrôler avec l'ordinateur le flux lumineux de la source éblouissante réfléchie par le miroir et le flux lumineux de la cible.

L'utilisation d'un ordinateur est particulièrement adaptée pour tester la vision centrale et les effets de l'éblouissement puisque les afficheurs des ordinateurs habituellement utilisés par l'homme du métier sont ouverts à un angle de 15-25°.

Le calibrage des dispositifs selon l'invention peut être effectué en utilisant des suspensions aqueuses de particules de latex. De préférence, les particules de latex ont un diamètre de 1,5 µm. On mesure la densité optique de chaque suspension latex par spectrophotométrie (in vitro). On positionne ensuite une personne non atteinte devant le dispositif selon l'invention, et on place devant son oeil successivement chaque suspension latex. On mesure $\omega$ et on note la relation entre la densité optique de la suspension (qui imite l'opacité de la lentille d'un patient atteint, à différents stades) et $\omega$.

## Revendications

1. Procédé d'évaluation de dysfonctionnements oculaires, caractérisé en ce qu'on place l'oeil (1) d'un patient, à une distance prédéfinie d'une source de lumière éblouissante (3), de manière à ce qu'il visualise une cible (4) placée à proximité de la source de lumière éblouissante (3), on approche la cible (4) de la source (3) jusqu'à ce que le patient ne puisse plus distinguer la cible (4) de la source (3), on éloigne ensuite lentement la cible (4) de la source (3), jusqu'à l'instant précis où le patient distingue à nouveau la cible (4) et, à cet instant, on mesure l'angle $\omega$ de la lumière incidente entre la source (3) et la cible (4).

2. Procédé d'évaluation de dysfonctionnements oculaires selon la revendication 1, caractérisé en ce que le patient, soumis le cas échéant à correction, est placé dans une pièce plongée dans la pénombre.

3. Procédé d'évaluation de dysfonctionnements oculaires selon l'une quelconque des revendications 1 ou 2, caractérisé en ce que ladite distance prédéfinie entre la source de lumière (3) et l'oeil (1) du patient est 300 mm.

4. Procédé d'évaluation de dysfonctionnements oculaires selon l'une quelconque des revendications 1 à 3, caractérisé en ce que ladite source de lumière éblouissante (3) est une lampe halogène ou une lampe à incandescence.

5. Procédé d'évaluation de dysfonctionnements oculaires selon l'une quelconque des revendications 1 à 4, caractérisé en ce que ladite cible (4) est lumineuse.

6. Procédé d'évaluation des opacités d'un milieu oculaire selon l'une quelconque des revendications 1 à 5, caractérisé en ce que l'on modifie artificiellement le contraste sur la cible (4).

7. Procédé d'évaluation des opacités d'un milieu oculaire selon la revendication 5, caractérisé en ce que ladite cible (4) est une source de lumière de couleur rouge ou verte.

8. Procédé d'évaluation des opacités d'un milieu oculaire selon l'une quelconque des revendications 1 à 7, caractérisé en ce que la source (3) et la cible (4) sont dans le même plan tangentiel vertical.

9. Procédé d'évaluation des opacités d'un milieu oculaire selon la revendication 8, caractérisé en ce que la mesure de l'angle de la lumière incidente entre la source (3) et la cible (4) s'effectue en mesurant la distance entre ladite source (3) et ladite cible (4).

10. Application du procédé selon les revendications 1 à 9 pour l'évaluation des opacités des milieux oculaires.

11. Application du procédé selon l'une quelconque des revendications 1 à 8 pour contrôler les fonctions rétiniennes.

12. Dispositif de mise en oeuvre du procédé selon l'une quelconque des revendications 1 à 8, caractérisé en ce qu'il comprend, à hauteur de l'oeil (1) du patient, une source de lumière éblouissante (3) et une cible (4) susceptibles de coulisser sur une réglette (11).

13. Dispositif selon la revendication 12, caractérisé en ce qu'il comprend en outre un tube conique ou cylindrique destiné à canaliser le champ de vision du patient.

14. Dispositif de mise en oeuvre du procédé selon l'une quelconque des revendications 1 à 8, caractérisé en ce qu'il est adapté sur un dispositif ophtalmologique de mesure de l'acuité visuelle soit simplement hémisphérique, soit à périmètre hémisphérique.

15. Dispositif selon la revendication 14, caractérisé en ce que ledit dispositif ophtalmologique de mesure de l'acuité visuelle à périmètre hémisphérique comprend un logiciel de mesure.

FIG. 1

FIG. 2

## EP 0 893 092 A1

Office européen
des brevets

**RAPPORT DE RECHERCHE EUROPEENNE**

Numéro de la demande

EP 98 48 0047

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int.Cl.6) |
| A | US 4 765 732 A (ENOCH) 23 août 1988<br><br>* abrégé *<br>* colonne 3, ligne 57 - colonne 4, ligne 20 *<br>* colonne 5, ligne 23 - colonne 11, ligne 17 *<br>* figures * | 1-3,5,6,<br>8-11 | A61B3/02<br>A61B3/06 |
| A | ENOCH ET AL.: "Hyperacuity test to evaluate vision through dense cataracts: research preliminary to a clinical study in India."<br>OPTICAL ENGINEERING,<br>vol. 34, no. 3, mars 1995, pages 765-771,<br>XP000495221<br>bellingham, us<br>* page 765, colonne de gauche, ligne 1 - page 768, colonne de gauche, ligne 25 *<br>* figures 1-4 * | 1,4,5,9,<br>10,12,15 | |
| A | US 4 764 007 A (TASK) 16 août 1988<br><br>* colonne 2, ligne 53 - colonne 3, ligne 53 *<br>* colonne 5, ligne 8 - ligne 37 *<br>* figures 1-4 * | 1,4,6,<br>10,12 | DOMAINES TECHNIQUES RECHERCHES (Int.Cl.6)<br><br>A61B |
| A | US 3 737 217 A (HAINES ET AL.) 5 juin 1973<br>* colonne 3, ligne 16 - colonne 4, ligne 37 *<br>* colonne 6, ligne 52 - colonne 7, ligne 40 * | 1,13 | |

Le présent rapport a été établi pour toutes les revendications

| Lieu de la recherche | Date d'achèvement de la recherche | Examinateur |
|---|---|---|
| LA HAYE | 3 novembre 1998 | Chen, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

8